(19) Europäisches Patentamt
European Patent Office
Office européen des brevets

(11) Veröffentlichungsnummer: **0 032 564**
**A1**

(12) **EUROPÄISCHE PATENTANMELDUNG**

(21) Anmeldenummer: **80108008.6**

(22) Anmeldetag: **18.12.80**

(51) Int. Cl.³: **C 07 D 241/44**, C 07 D 473/08, C 07 D 473/10, C 07 D 473/12, A 61 K 31/495, A 61 K 31/52

(30) Priorität: **28.12.79 JP 173249/79**
**29.01.80 CH 717/80**
**11.12.80 CH 9144/80**
**28.12.79 JP 173248/79**

(43) Veröffentlichungstag der Anmeldung: **29.07.81**
**Patentblatt 81/30**

(84) Benannte Vertragsstaaten: **AT BE CH DE FR GB IT LI LU NL SE**

(71) Anmelder: **MEDICHEMIE AG, Brühlstrasse 50, CH-4107 Ettingen/Basel (CH)**

(72) Erfinder: **Widauer, Josef Olaf, Dr. med., Beim Lindenbaum 17, CH-4123 Allschwil (CH)**
Erfinder: **Hoeriger, Niklaus, Dr., Hackbergstrasse 61, CH-4125 Riehen (CH)**
Erfinder: **Hara, Hiroto, 13-2, Ogawa 1-chome Machida-shi, Tokyo (JP)**
Erfinder: **Narimatsu, Akihiro, 26-7, Sakuradai Midori-ku, Yokohama-shi (JP)**
Erfinder: **Egawa, Mitsuo, 5-1, Tsutsujigaoka Midori-ku, Yokohama-shi (JP)**

(74) Vertreter: **Fiedler, Otto Karl, Dipl.-Ing., Rheinhöhe 9, D-7891 Küssaberg 1 (DE)**

(54) **Pharmazeutische Verwendung von Caroverin und/oder Caroverinsalzen sowie Caroverinsalze mit Xanthinderivat-Säureresten und Nicotinsäurerest.**

(57) Pharmazeutische Verwendung von Caroverin der Formel:

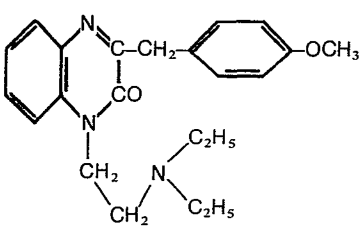

pharmazeutisch verträgliche Caroverinsalze, insbesondere Caroverinfumarat, Caroverinsalze mit Xanthinderivat-Säurerest, insbesondere Caroverin-Theophyllinat, -Coffeinat, -Theobrominat, sowie Caroverin-Nicotinat als neue Substanzen, insbesondere für pharmazeutische Verwendung.

**EP 0 032 564 A1**

Pharmazeutische Verwendung von Caroverin und/oder Caroverinsalzen sowie Caroverinsalze mit Xanthinderivat-Säureresten und Nicotinsäurerest.

Die Erfindung bezieht sich auf pharmazeutische Mittel mit Caroverin und/oder Caroverinsalzen, insbesondere Caroverinfumarat, und auf die pharmakologische Verwendung dieser Substanzen.

Caroverin ist die internationale, freie Kurzbezeichnung für eine organische Base mit der Formel: 1-(2-diäthylaminoäthyl)-3-(p-methoxybenzyl)-1,2-dihydroquinoxalon-2.

Die Strukturformel lautet:

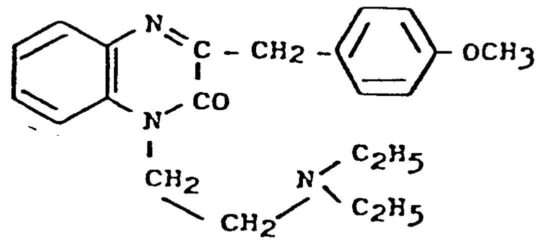

Caroverin ist als spasmolytischer Wirkstoff (auch unter der Bezeichnung "Spasmium" im Handel) sowie mit der Indikation zur Verbesserung der celebralen Durchblutung bekannt (Subsidia Medica, Sonderheft Jahrg. 22, Heft 3, 1970, Verlag Brüder Hollinek,Wien).

Weiterhin ist mit dem gleichen pharmakologischen Effekt das Fumarsäuresalz dieser Base bekannt (japanische Offenlegungsschrift 260 410/5.

Die Strukturformel lautet:

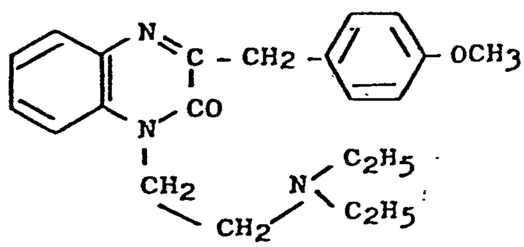

·

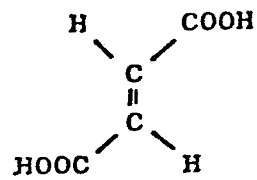

Durch pharmakologische Untersuchungen und Versuchsreihen wurde nun festgestellt, dass Caroverin und insbesondere Caroverinfumarat eine hemmende Wirkung bezüglich der Blutplättchenaggregation (Thrombozytenaggregation) aufweist. Darüberhinaus wurde festgestellt, dass die genannten Substanzen eine markante Erhöhung der arteriellen Durchblutung bewirken, insbesondere der Coronardurchblutung, und für die Behandlung von ischämischen Herzkrankheiten, insbesondere der angina pectoris und des Myocardinfarktes, indiziert sind. Insbesondere führt auch der kombinierte Effekt von Aggregationshemmung und Förderung der Arteriendurchblutung zu interessanten Indikationen.

Caroverin und Caroverinfumarat können grundsätzlich allein, vorzugsweise jedoch in Kombination mit pharmazeutisch verträglichen Trägersubstanzen verabreicht werden. Die Dosierung ist abhängig vom allgemeinen Gesundheitszustand und Gewicht des Patienten sowie von den speziellen Bedingungen der zu behandelnden Krankheit, von etwa angewendeten, gleichlaufenden Behandlungen, von der Verabreichungsfrequenz und der speziellen Art des erstrebten Effektes. Die therapeutische Dosierung beträgt bei parenteraler

Verabreichung im allgemeinen 0,3 bis 2 mg/kg pro Tag, bezogen auf das Patientengewicht, sowie 2 bis 10 mg/kg pro Tag bei oraler Verabreichung.

Caroverinfumarat kann in Form von festen Partikeln, etwa Tabletten, Kapseln, Pulverpackungen und dergleichen, in Form flüssiger Lösungen für orale Verabreichung oder in Form von sterilen Flüssigkeitszubereitungen wie Lösungen oder Suspensionen für parenterale Verabreichung angewendet werden. In solchen Zusammensetzungen sind neben dem Wirkstoff feste oder flüssige, ungiftige pharmazeutische Trägersubstanzen enthalten. In einer praktischen Ausführungsform wurde eine Gelatine-Trägerkapsel angewendet, in einer anderen Ausführungsform wurde der Wirkstoff tabletiert oder granuliert und gegebenenfalls mit geeigneten Zuschlagstoffen versehen, oder aber in ein Pulverpaket verbracht. Als Trägersubstanzen mit Transportfunktion (Vehikel) für Zusammensetzungen der vorgenannten Art kommen in Betracht: Wasser, Gelatine, Saccharide wie Lactose und Glucose, Stärkepräparate aus Korn, Weizen, Reis und Pfeilwurzel, Fettsäuren wie Stearinsäure, Fettsäuresalze wie Calziumstearat und Magnesiumstearat, Talcum, vegetabile Oel, Alkohole wie Stearylalkohol und Benzylalkohol, Gummi, Polyalkylglykole, Zellulosederivate, Polyvenylpyrolidon (PVP), Casein-Formaldehyd und Metacrylsäurederivate.

Die Medikamente in Form von Kapseln, Tabletten, Granulaten und Pulvern enthalten im allgemeinen zwischen 5 bis 100 Gew. %, vorzugsweise zwischen 25 und 100 Gew. %, an Wirksubstanz.

Als pharmazeutische Trägersubstanz kommen Flüssigkeiten wie Wasser und Oele einschliesslich Mineralöle sowie tierische, pflanzliche und synthetische Oele in Betracht, beispielsweise auch Erdnussöl, Soiaöl, Sesamöl und dergleichen. Im allgemeinen sind wässrige Salzlösungen, wässrige Dextroselösungen und ähnliche Zuckerlösungen, fernerGlykole wie Aethylenglycol, Propylenglycol und Polyäthylenglycol zu bevorzugen.

Caroverinfumarat kann parenteral durch intramuskuläre, intravenöse oder subcutane Injektion verabreicht werden. Hierfür kommen sterile Lösungen mit zusätzlichen gelösten Substanzen in Betracht, beispielsweise Salze oder Glukosen, zur Einstellung von isotonischen Lösungen.

Beispiele von zur Injekion geeigneten Lösungsmitteln sind steriles Wasser, Lidocain-Hydrochloridlösung (für intramuskuläre Injektion), wässrige Salzlösungen, Glucoselösungen (für intravenöse Injektion) und Lösungen mit Elektrolyten (für intravenöse Injektion). Solche Injektionslösungen enthalten im allgemeinen von 0,5 bis etwa 20 Gew.%, vorzugsweise zwischen 1 und etwa 10 Gew.%, der Wirksubstanz.

Zur oralen Verabreichung kommt eine geeignete Suspension oder ein Sirup in Betracht, wobei die Wirksubstanz typisch in einem Anteil von etwa o,5 bis 10 Gew.% vorhanden ist. Als pharmazeutische Trägersubstanz für solche Zusammensetzungen kommen wasserhaltige Flüssigkeiten in Betracht, beispielsweise aromatisiertes Wasser, Sirup oder ein pharmazeutisch geeigneter Schleimstoff.

Aus den Experimentaluntersuchungen, mittels deren die oben angeführten, erfindungsgemässen Effekte und Indikationen festgestellt bzw. erhärtet wurden, seien die folgenden Beispiele zur näheren Erläuterung der Erfindung dargelegt.

## Beispiel 1

## Versuch zur Blutplättchen-Aggregationshemmung

Thrombozytenreiches Plasma wurde aus citriertem Kaninchenblut gewonnen. Die Thrombozytenaggregation wurde durch Collagen aus Rinder- Achillessehnen induziert, worauf der Aggregations-Hemmeffekt durch optische Trübungsmessung (Turbidimetrie) untersucht und quantifiziert werden konnte.

Als Vergleichs-Wirksubstanzen neben Caroverinfumarat wurden Benzyclanfumarat und Aspirin verwendet.

Die Versuchsergebnisse sind in den Messdiagrammen der Fig. 1 bis 3 wiedergegeben, wobei es sich um typische Resultate handelt.

Bei diesen Diagrammen handelt es sich um Aufnahmen des zeitlichen Verlaufes einer optischen Messgrösse als Mass eines durch das Präparat tretenden Lichtanteils, entsprechend einem reziproken Mass für den Agglutinations- oder Aggregationsgrad. Die verschiedenen Messkurven für je eine Wirksubstanz entsprechen unter-

schiedlichen molaren Konzentrationen in Mikromol des Wirkstoffes als Parameter. Die erste Messkurve in Fig. 1 entspricht einem Kontroll-Leerversuch.

Der Vergleich der Ergebnisse für Caroverinfumarat mit denjenigen für Benzyclanfumarat und Aspirin zeigt einen eindeutigen Hemmungseffekt für Caroverinfumarat mit einer auf die Konzentration bezogenen Intensität im Bereich zwischen den beiden letztgenannten Vergleichssubstanzen.

Aehnliche Ergebnisse wurden mit der gleichen Versuchsmethode an Gerinnungspräparaten von menschlichem Blut erzielt.

Beispiel 2

Versuch zur Erhöhung der Coronardurchblutung

Es wurde der coronare Sinus-Ausfluss(CSF), der System-Blutdruck (SEP) und die Herzfrequenz (HR) an Hunden untersucht. Für den coronaren Sinus-Ausfluss wurde die in Clin. Pharmacol. Physiol., 5, 107 - 115, 1978, Australien,beschriebene Untersuchungsmethode angewendet.

Zeitdiagramme der genannten Messgrössen SBP (mmHg), CSF (ml/min) und HR ($min^{-1}$) wurde für vier verschiedene Dosierungen von Caroverinfumarat, nämlich 0,03, 0,1, 0,3, 1,0 und 3,0 mg/kg

(bezogen auf das Körpergewicht) aufgenommen. Die Diagramme für die drei erstgenannten Dosierungswerte sind in Fig. 4 und diejenigen für die beiden letztgenannten Dosierungswerte in Fig. 5 bzw. Fig. 6 wiedergegeben. Der Zuführungszeitpunkt der Wirksubstanz ist in den Diagrammen gemäss Fig. 4 durch das Zeichen "o" markiert, während in den Diagrammen gemäss Fig. 5 und 6 das Zuführungsintervall jeweils durch einen Horizontalstrich unterhalb der CSF-Messkurve angegeben ist.

Die Messkurven zeigen deutlich folgende, in ihrer Intensität mit der Dosierung zunehmende Effekte:

1. Herabsetzung des System-Blutdrucks,
2. Herabsetzung der Herzfrequenz und
3. Rasch zunehmende und langsam abklingende Zunahme des coronaren Sinus-Ausflusses.

Beispiel 3

Untersuchung der akuten Toxizität an männlichen Mäusen

Es wurden folgende Standard-Grenzwerte $LD_{50}$ (mg/kg) ermittelt:

Intravenöse Zuführung ...................... 39
Intraperitoneale Zuführung ................. 138
Orale Zuführung ............................ 800.

Zum Erfindungsgegenstand gehört ferner die Verwendung von Caroverin bzw. Caroverinsalzen, insbesondere Caroverinfumarat, zur Behandlung von erhöhtem Blutdruck und tachycarden Herzrhythmusstörungen bzw. zur Herstellung entsprechender pharmazeutischer Mittel. Der entsprechende Effekt der Herabsetzung von Blutdruck und Herzfrequenz ergibt sich bereits aus den Experimentaluntersuchungen gemäss Fig. 4 bis 6 neben der Erhöhung der Coronardurchblutung. Zur Erhärtung des frequenzabsenkenden Effektes seien insbesondere noch folgende Tierversuche angeführt:

Untersuchung
über die Wirkung von Caroverin auf das isolierte, nach Langendorff perfundierte Meerschweinchenherz, im Vergleich zu Theophyllin

**Beispiel 4**
**Vergleichsversuche Caroverin/Theophyllin zur Herzfrequenzabsenkung und Coronardurchblutung.**

Meerschweinchen wurden durch Genickschlag getötet, der Thorax eröffnet, der Herzbeutel aufgeschnitten, die Aorta mit einem Faden angeschlungen und in der pars ascendens angeschnitten. Nach Einbinden einer Glaskanüle in die Aorta wurde die aorta pulmonalis eröffnet und anschliessend das Coronargefässsystem durch die Aortenkanüle unter leichtem Druck mit einer Spitze durchgespült. Nach Durchtrennung aller anhängenden Gefässe wurde das Herz an der Aortenkanüle in die feuchte Kammer einer modifizierten Langendorff-Apparatur gehängt und mit carbogengesättigter Tyrodelö-

sung von 37°C mit einem Druck von 55 cm $H_2O$ perfundiert. An der Herzspitze wurde eine Serre fine angebracht, die über einen Faden mit einem zweiarmigen Hebel verbunden war, der die Kontraktionsamplitude auf einem Russkymographen aufzeichnete. Alle Herzen waren mit 4 g belastet. Der Schreibhebel passierte bei seinen Ausschlägen die Lichtschranke einer Fotozelle. Dadurch konnte die Herzfrequenz über einen Totalisator (nach FLEISCH, Firma B. Braun, Melsungen) registriert werden. Die aus dem rechten Vorhof frei abfliessende Perfusionslösung tropfte auf einen elektrischen Kontakt. Die Tropfenzahl wurde mittels eines zweiten Totalisators aufgezeichnet, und nach Eichung in ml/min der Coronardurchfluss bestimmt.
Nach Fertigstellung der Präparate wurde bis zum Versuchsbeginn 45 Min. gewartet. Nach dieser Zeit hatten Kontaktionsamplitude, Frequenz und Coronardurchblutung ein konstantes Niveau erreicht.

Versuchsablauf

Caroverin wurde in Einzeldosen von 1, 10, 100 sowie 1000 ug verabreicht. Theophyllin wurde in Dosen von 10, 100 sowie 1000 ug gegeben. In allen Versuchen wurden Caroverin und Theophyllin abwechselnd verabreicht, wobei jeweils eine Zeitspanne von 5 Min. zwischen zwei Einzelinjektionen lag. Alle Injektionsvolumina betrugen 0.1 ml.

In der beigefügten Tabelle sind die mittleren Ausgangshöhen von Coronarfluss und Kontraktionsamplitude ± mittlere Fehler der Mittelwerte angegeben. Die Effekte der Substanzen wurden ermittelt, indem in jedem Versuch bei jeder Einzeldosis die maximalen

Zu- bzw. Abnahmen der entsprechenden Parameter gegenüber dem jeweiligen Ausganswert bestimmt und diese Einzeleffekte gemittelt wurden. Statistische Signifikanzberechnungen erfolgten nach dem t-Test (student's-Test).

Ergebnisse (Fig. 7, Tabelle I,II)

Caroverin hatte eine dosisabhängige, coronardurchflusssteigernde Wirkung. Theophyllin hatte ebenfalls eine steigernde Wirkung auf den Coronardurchfluss, wobei die Wirkung von 1000 µg etwa derjenigen von 100 µg Caroverin entsprach.

100 µg Caroverin verminderte die Kontraktionsamplitude signifikant ($p < 0.01$). Theophyllin verursachte in den angewandten Dosen keine signifikanten Aenderungen der Kontraktionsamplitude.

In allen Versuchen hatte Caroverin in Dosen von 100 µg einen langanhaltenden frequenzsenkenden Effekt, während Theophyllin in der Dosis von 1000 µg in alllen Versuchen die Herzfrequenz vorübergehend steigerte.

Effekt und Indikation wurden ferner durch folgende klinischen Versuche sichergestellt:

Beispiel 5

Versuche über den Effekt von Caroverinfumarat auf Blutdruck und Herzfrequenz am Menschen

Bei insgesamt 6 Patienten (5 ♀, 1 ♂) mit Arrhythmia absoluta wurden innerhalb 3 - 5 Min. 200 mg Caroverinfumarat i.v. verabreicht. Bei 4 der Patienten handelte es sich um eine wahrscheinlich kardio-sklerotisch bedingte Arrhythmia absoluta, bei 2 Patienten lag ein Mitralvitium zugrunde. Mittleres Alter der Probanden war 65 (+6/-11) Jahre. Registriert wurden EKG (Frequenz) und Blutdruck (Riva-Rocci) bis max. 13 Minuten nach Injektionsbeginn. Die Min.-Frequenz ermittelte sich aus 10 aufeinanderfolgenden QRS-Komplexen.

In den Tabellen III bis VI gemäss Figuren 8 bis 11 sind Blutdruck und Frequenz eingetragen und zeigen deren Verhalten während und nach der Injektion.

Ergebnis

Der maximale Frequenz-Abfall im Vergleich zum Ausgangswert beträgt im Mittel 26,6 (+ 7,2/-4,7) %. Der maximale systolische Blutdruckabfall zeigt 29,3 (+ 8,2/-9,3) %; der diastolische Blutdruckabfall im Mittel 26,6 (+ 10,9/-11,6) %. Die Vorhofflimmer-Frequenz verbleibt ohne messbare Veränderung; ein rhythmisierender Effekt wurde nicht nachgewiesen.

Nebenwirkungen

Bei 2 Patienten wurde während der Injektion subjektive Beschwerden in Form von Kopfdruck, Hitzegefühl und Ohrensausen angegeben, die maximal ca. 45 Min. anhielten.

Die Resultate zeigen, dass Caroverin zur Behandlung der genannten Krankheiten geeignet ist. Das Medikament kann enteral oder parenteral verabreicht werden. Die Dosis beträgt zweckmässig etwa 40 bis 400 mg für einen erwachsenen Menschen.

Ergänzend seien noch folgende klinischen Untersuchungen zum herzfrequenzabsenkenden Effekt von Caroverinfumarat wiedergegeben:

Patientenwahl

a) Anzahl: je 6 Patienten der Gruppen A, B und C.
Die Probandenzahl der Gruppe D hängt davon ab wie viele geeignete Patienten sich ad hoc finden.

b) Die Patientenauswahl erfolgt nach den festgelegten Kriterien des Prüfers.

c) Kontraindikationen
Antiarrhythmika, Antipektanginosa.
Alle Patienten, die nicht für eine klinische Studie mit Ca-Antagonisten geeignet sind, oder unter Krankheiten leiden, die die Resultate verfälschen könnten.

Methode

Die Patienten werden im Liegen nach Anlegen eines Venenkatheters

laufend kontrolliert.

Leerbefunde 10 und 5' vor Beginn der Medikation.

Dann Befundnahme bei Initialdosis von 20 mg i.v.

Weitere Befundnahmen in 4-minütigen Abständen bei Dosen von je 10 mg i.v., bis zu einer Maximaldosis von 120 mg i.v.

Nach Beendigung der Medikation, Nachkontrolle in 10-minütigen Abständen bis ca. 4 Stunden nach Beginn der Medikation.

Abbruch der Medikation bei:

1) AV-Block 1. Grades oder mehr
2) Blutdruckabfall systolisch mehr als 30 mmHg
3) Dyspnoe
4) Bei Patienten der Gruppe D sobald der therapeutische Effekt erreicht ist.

Parameter

Diagnose

Alter

Geschlecht

Gewicht

zusätzliche Therapie, Vorbehandlung

EKG

Herzfrequenz

Blutdruck systolisch/diastolisch

PQ-Zeit

QRS-Dauer

QT-Zeit

ST-Senkung

Doppler-Druck-Messung peripher

Ergebnisse

In einem ersten Schritt wurden die Zeitreihen (Herzfrequenz-Mittelwerte und Streuungen) je Behandlungsgruppe einzeln berechnet und graphisch dargestellt (s. Abbildung und Tabellen 1. im Anhang). Es ergaben sich Verläufe, die anzeigen, dass sich die Behandlungsgruppen sowohl bezüglich ihrer Vorwerte (Mittelwerte) als auch bezüglich ihrer Verläufe während der 40-minütigen Applikation und der 3 Stunden Nachbeobachtung unterscheiden. Es ist ebenfalls festzuhalten, dass die Werte zu Beginn der Applikation gegenüber den Vorwerten durchwegs erhöht sind.

Um die Herzfrequenz-Verläufe innerhalb der Behandlungsgruppen im Sinne des Auswertungszieles besser charakterisieren zu können, wurden (screeningmässig) die Differenzen der Mittelwerte zu jedem Beobachtungszeitpunkt nach Applikationsbeginn sowohl zu den Vorwerten als auch zum Wert bei Applikationsbeginn dargestellt und mittels t-Test-Statistiken - unter Vernachlässigung der Eigenschaften abhängiger Zeitpunkte und der Mehrfachtests - auf Signifikanz geprüft.

Kurvenverläufe und Signifikanzergebnisse lassen für alle drei Behandlungsgruppen charakteristische und unterschiedliche Verläufe vermuten:

- <u>Herzgesunde Probanden</u> (Behandlungsgruppe A)
  Ausgehend von Herzfrequenzen um 72/Min. vor Applikation (100 %) wird zum Ende der Applikation nach 40 Min. eine mittlere Herzfrequenz von 62/Min. (86 %) beobachtet, die bis 2 Std. nach Applikationsbeginn weiter sinkt auf einen Bereich von 57/Min. (79 %) und dann bis zum Ende der 4 Std. Beobachtungszeit anhält.

- <u>Patienten mit koronarer Herzkrankheit</u> (Behandlungsgruppe B)
  Ausgehend von Herzfrequenzen um 77/Min. vor Applikation (100 %) wird zum Ende der Applikation eine mittlere Herzfrequenz von 69/Min. (90 %) erreicht, die bis ca. 70 Min. nach Applikationsbeginn auf 65/Min. (84 %) weiter sinkt und sich bis zum Ende der Applikationszeit im Bereich zwischen diesen beiden Werten weiterbewegt.

- <u>Patienten mit krankem Reizleitungssystem</u> (Behandlungsgruppe C)
  Ausgehend von Herzfrequenzen um 66/Min. vor Applikation (100 %) wird zum Ende der Applikation eine mittlere Herzfrequenz von 57/Min. (86 %) beobachtet, die dann im Zeitraum von 2 1/2 bis 3 Std. nach Applikationsbeginn auf mittlere 54/min. (82 %) weiter absinkt. Die weiteren mittleren Beobachtungswerte bewegen sich dann bis zum Beobachtungsende nach 4 Stunden wiederum im Bereich zwischen diesem Minimum und dem Applikationsendwert.

Vergleichend kann vermutet werden, dass sich die Wirkung auf die Herzfrequenz bei herzgesunden Probanden (A) und bei den Patienten mit krankem Reizleitungssystem (C) während der Applikation klarer manifestiert hat als in der Gruppe der Patienten mit koronarer Herzkrankheit (B).

Fig. 11a zeigt die Ergebnisse der Untersuchung in Form von Messdiagrammen der Herzfrequenz über der Zeit.

Ebenfalls zum Erfindungsgegenstand gehört die Verwendung von Caroverin bzw. Caroverinsalzen, insbesondere Caroverinfumarat, in pharmazeutischen Mitteln mit einem calcium-antagonistischen Wirkungsspektrum. Hiervon wurden umfangreiche Experimentaluntersuchungen durchgeführt.

Hiervon seien die Versuchsreihen gemäss den Diagrammen in Fig. 12, 13, 14 und 15 angeführt und wie folgt erläutert:

Beispiel 6 (Fig. 12)

Gleichzeitige Registrierung der intrazellulär abgeleiteten Aktionspotentiale und der isometrischen Mechanogramme von einem isolierten Meerschweinchen-Papillarmuskel unter dem Einfluss steigender Konzentrationen von Caroverin (5; 10 und 20mg/l) in Tyrodelösung. Die Aktionspotentiale wurden während eines 180 Minuten dauernden Einstichs der Mikroelektrode an ein und derselben Myocardfaser gewonnen. Die einzelnen Konzentrationen kamen jeweils für die Dauer von 40 Minuten zur Anwendung.

Beispiel 7 (Fig. 13)

Hemmungen der $K^+$-Kontraktur isolierter Coronararterienstreifen vom Schwein durch steigende Konzentrationen von Caroverin (o,05; 0,1 und 0,5 mg/l). Partielle Restitution der mechanischen Spannungsentwicklung durch Extra-Calcium.

Beispiel 8 (Fig. 14)

Hemmung der $K^+$-Kontraktur isolierter Coronararterien-Streifen vom Schwein durch steigende Konzentrationen von Caroverin (1,0; 2,0 und 5,0 mg/l)= Partielle Restitution der mechanischen Spannungsentwicklung durch Extra-Calcium in Anwesenheit von 1 mg Caroverin/Liter..

Beispiel 9 (Fig. 15)

Neutralisation der tonussteigernden Effekte einer höheren Konzentration von k-Strophanthin an der glatten Coronar-Muskulatur eines Schweines durch Caroverin. Teilweise Restitution des Coronartonus durch Steigerung der extra-cellulären $Ca^{++}$-Konzentration.

Ergebnis

In den durchgeführten Experimenten erweist sich Caroverin als spezifischer $Ca^{++}$-Antagonist mit sehr stark akzentuierter Gefässwirkung. An elektrisch gereizten isolierten Meerschweinchen-Papillarmuskeln blockierten 10 mg Caroverin/l den $Ca^{++}$-Einstrom während der Erregung zu etwa 50 %, ohne die transmembranären $Na^+$- und $K^+$-Fluxe zu reduzieren (Fig. 12). Etwa im gleichen Konzentrationsbereich hemmt Caroverin die elektrischen Parameter $Ca^{++}$-getragener Aktionspotentiale (AP-Amplitude, max. Aufstrichsteilheit des Ap, AP-Dauer) und die zugehörigen Mechanogramme. Demgegenüber lässt sich eine etwa 50 %ige elektromechanische Entkoppelung $K^+$-depolarisierter Coronararterienstreifen schon mit

0,25 mg Caroverin/l herbeiführen (Fig. 13 und 14). Die Dosiswirkungsbeziehungen für die Unterdrückung glykosid-bedingter Coronarspasmen liegen etwa im gleichen Bereich wie diejenigen für die Hemmung der $K^+$-Kontraktur (Fig. 15).

Zum Erfindungsgegenstand gehören ferner gewisse spezielle Caroverinsalze, die bisher auch als chemische Substanzen noch nicht bekannt waren, insbesondere also auch nicht für pharmakologische Verwendung. Es handelt sich hierbei um Caroverinsalze mit einem sauren Xanthinderivat als Säurerest, insbesondere um Caroverin-Theophyllinat,Caroverin-Theobrominat und Caroverin-Coffeinat. Es handelt sich hierbei um pharmakologisch eigenständige Substanzen, für die erfindungsgemäss insbesondere eine gefässerweiternde und blutdrucksenkende sowie vor allem überraschend eine diuretische und eine bronchodilatatorische Wirkung festgestellt werden konnte.

Weiterhin gehört zum Erfindungsgegenstand ein Caroverinsalz mit Nicotinsäurerest, also Caroverin-Nicotinat, ebenfalls eine chemisch neue und pharmakologisch eigenständige Substanz. Hierfür wurde erfindungsgemäss eine betonte gefässerweiternde und eine diuretische sowie auch eine thrombozyten-aggregationshemmende Wirkung ermittelt. Diese neue Substanz eignet sich insbesondere zur Behandlung von Durchblutungsstörungen und insbesondere zur prophylaktischen Behandlung der Arteriosklerose.

Für die klinische Anwendung dieser neuen Pharmaka ist eine parenterale Verabreichung der oralen vorzuziehen, weil die genannten

Salze bei der Magenpassage Spaltung und Umsetzung in Hydrochlorid unter dem Einfluss der Magensalzsäure erleiden können.

Abschliessend werden zur pharmakologischen Wirkung von Caroverin-Theophylinat und Caroverin-Nicotinat folgende Ergebnisse von Untersuchungen in vivo und in vitro wiedergegeben:

1. Caroverin-Theophyllinat

a) Akute Toxizität an der Maus

300 mg/kg p.os ergaben keine Auffälligkeiten. Letale Dosis 200 mg/kg i.p.. Nach 100 mg/kg i.p. ergab sich eine leichte Muskelrelaxation.

b) Diuretischer Effekt

Bei einer verabreichten Dosis von 20 mg/kg p.os ergab sich ein diuretischer Wirkungs-Score von 2.8 (Vergleich: 20 mg/kg Spironolacton als Referenzsubstanz ergab einen entsprechenden Score von 3.6).

Diese Untersuchung an Ratten wurde nach der Methode von Lipschitz et al., j.p.e.t. 79, 97-110, 1954, durchgeführt.
Der errechnete Score entspricht einem Quotienten aus Na-Ausscheidungen nach der Testsubstanz durch Na-Ausscheidung des Kontroll-Leerwertes.

c) Bronchodilatatorische Wirkung

In vitro ergab sich am Meerschweinchen-Lungenpräparat mit 100 µg/ml eine eindeutige Zunahme der Perfusions-Fliessrate.

d) Anticholinergische Wirkung

In vitro ergab sich am Meerschweinchen mit einer Dosis von 10 µg/ml eine mehr als 80 %ige Hemmung des verwendeten Agonisten (Atropin).

2. Caroverin-Nicotinat

a) Akute Toxizität an der Maus

300 mg/kg p.os ergaben keine Auffälligkeiten. Letale Dosis: 200 mg/kg i.p.. Nach 100 mg/kg i.p. ergab sich eine Abnahme des Muskeltonus in den Extremitäten, sowie eine leichte Abnahme des Haltegriffes.

b) Collagen-induzierte Plättchen-Aggregation

In vitro ergab sich am Kaninchen mit 10 mcg/ml eine 100 %ige Hemmung der collagen-induzierten Plättchenaggregation.

c) Diuretischer Effekt

Bei einer verabreichten Dosis von 20 mg/kg p.os ergab sich ein diuretischer Wirkungswert von 2.6 (Vergleich: 20 mg/kg Spirolocton als Referenzsubstanz ergab einen entsprechenden Score von 3.6).

Die Untersuchung an Ratten wurde wie unter 1. durchgeführt. Für den diuretischen Wirkungs-Score gilt gleiches.

d)Bronchodilatatorische Wirkung

In vitro ergab sich am Meerschweinchen-Lungenpräparat mit 100 µg/ml eine eindeutige Zunahme der Perfusions-Fliessrate.

e) Anti-Cholinergische Wirkung

In vitro ergab sich am Meerschweinchen mit einer Dosis von 10 µg/ml eine mehr als 80 %ige Hemmung des verwendeten Agonisten (Atropin).

Zum Erfindungsgegenstand gehört ebenso die Verwendung von Caroverin und/oder pharmazeutisch verträglichen Caroverinsalzen, insbesondere Caroverinfumarat, als zentral wirkendes Muskelrelaxans.

Caroverin und seine pharmakologisch erträglichen Salze sind demgemäss verwendbar zur Behandlung von verschiedenen spastischen Paralysen, insbesondere solchen, die durch cerebrale Gefässkrankheiten verursacht sind, sowie zur Behandlung von schmerzhaften, mit Bewegungsstörungen verbundenen Spasmen wie die Lumbodorsodynie, Nacken-Oberarm-Syndrom, Schulter-Periarthritis und spondylosis deformans. Die folgenden Untersuchungsergebnisse sollen die betreffenden Erfindungsmerkmale näher erläutern:

Zum Hemmungseffekt der anemischen Decerebrationsstarre (Enthirnungsstarre)

Die Untersuchungen wurden ausgeführt nach der Methode von Fukuda et al. (Japanese Journal of Pharmacology, Band 24, Seiten 810 - 813, 1974).

Es wurden männliche Wister-Ratten mit einem Köpergewicht von 280 - 380 g verwendet. Die Tiere wurden mit Aether betäubt und am Rücken befestigt. Nach Freilegen der Luft- und Speiseröhre sowie der beiderseitigen, gemeinsamen Carotisarterien wurde die Luftröhre kanüliert und die Speiseröhre abgetrennt. Das Hinterhauptbein wurde durch Abschaben der anhängenden Muskulatur freigelegt. Nach beidseitigem Abbinden der gemeinsamen Carotisarterien wurde im mittleren Teil des Hinterhauptbeines eine Oeffnung von 5 mm Durchmesser trepaniert, um die Basilararterie sichtbar zu machen. Die dura mater wurde längs der Basilararterie

durchschnitten und letztere mit einem Coagulator ausgeätzt. Unmittelbar nach Beendigung dieser Massnahmen wurde die Anästhesie unterbrochen. Innerhalb von 30 min entwickelte sich Starre, und es trat eine deutliche Streckung der Extremitäten, des Nackens und des Schwanzes ein. Die Streckung der Vordergliedmassen wurde mit einem Dehnungsmessstreifen erfasst. 50 min nach Erholung von der Betäubung wurden in Salzlösung aufgelöste Pharmaka durch eine in die Oberschenkelvene eingeführte Kanüle intravenös zugeführt. Die verwendeten Pharmaka waren Caroverinfumarat und Referenz-Agenzien, nämlich Mephenesin und Tolperison-Hydrochlorid. Das Ergebnis nach Zuführung von 2 mg/kg Caroverinfumarat sind in der nachstehend eingefügten Tabelle wiedergegeben.

| | Dosis für 50%ige Inhibierung (mg/kg i.v.) | Zeit für 50%ige Erholung (min.) |
|---|---|---|
| Caroverinfumarat | 1.5 | > 30 |
| Mephenesin | 25.0 | ca 13 |
| Tolperison-Hydrochlorid | 4.0 | ca. 10 |

Die Ergebnisse zeigen, dass die entwickelte anemische Decerebrationsstarre durch Caroverinfumarat unverzüglich aufgehoben wurde und dass die Wirkung von Caroverinfumarat im Vergleich zu Tolperison-Hydrochlorid und Mephenesin stärker sowie länger andauernd war.

Toxizitätsuntersuchung an männlichen Mäusen

| Coroverinfumarat | $LD_{50}$ (mg/kg) |
|---|---|
| i. v. | 39 |
| i. p. | 138 |
| p.o. | 800 |

Fig. 16 zeigt ein Messdiagramm einer Starrheits-Messgrösse in p (Ordinate) über der Versuchszeit (Abszisse). Zur Veranschaulichung des Inhibitionseffektes von Caroverinfumarat gegenüber anemischer Decerebrationsstarre.

## Patentansprüche

1. Verwendung von Caroverin und/oder pharmazeutisch verträglichen Caroverinsalzen, insbesondere Caroverinfumarat zur Hemmung der Plättchenaggregation in menschlichem oder tierischem Blut.

2. Verwendung von Caroverin und/oder pharmazeutisch verträglichen Caroverinsalzen, insbesondere Caroverinfumarat zur Herstellung von pharmazeutischen Mitteln zur Hemmung der Blutplättchenaggregation.

3. Verwendung von Caroverin und/oder pharmazeutisch verträglichen Caroverinsalzen, insbesondere Caroverinfumarat zur Erhöhung der arteriellen Durchblutung.

4. Verwendung von Caroverin und/oder pharmazeutisch verträglichen Caroverinsalzen, insbesondere Caroverinfumarat zur Herstellung von pharmazeutischen Mitteln zur Erhöhung der arteriellen Durchblutung.

5. Verwendung von Caroverin und/oder pharmazeutisch verträglichen Caroverinsalzen, insbesondere Caroverinfumarat zur Erhöhung der Coronardurchblutung.

6. Verwendung von Caroverin und/oder pharmazeutisch verträglichen Caroverinsalzen, insbesondere Caroverinfumarat zur Herstellung von pharmzeutischen Mitteln zur Erhöhung der Conorardurchblutung.

7. Verwendung von Caroverin und/oder pharmazeutisch verträglichen Caroverinsalzen, insbesondere Caroverinfumarat zur Behandlung von ischämischen Herzkrankheiten.

8. Verwendung von Caroverin und/oder pharmazeutisch verträglichen Caroverinsalzen, insbesondere Caroverinfumarat zur Herstellung von pharmazeutischen Mitteln für die Behandlung ischämischer Herzkrankheiten.

9. Verwendung von Caroverin und/oder pharmazeutisch verträglichen Caroverinsalzen, insbesondere Caroverinfumarat zur Behandlung von angina pectoris.

10. Verwendung von Caroverin und/oder pharmazeutisch verträglichen Caroverinsalzen, insbesondere Caroverinfumarat

zur Herstellung von pharmazeutischen Mitteln für die Behandlung von angina pectoris.

11. Verwendung von Caroverin und/oder pharmazeutisch verträglichen Caroverinsalzen, insbesondere Caroverinfumarat zur Behandlung des Myocardinfarktes.

12. Verwendung von Caroverin und/oder pharmazeutisch verträglichen Caroverinsalzen, insbesondere Caroverinfumarat zur Herstellung von pharmazeutischen Mitteln für die Behandlung des Myocardinfarktes.

13. Verwendung von Caroverin und/oder pharmazeutisch verträglichen Caroverinsalzen, insbesondere Caroverinfumarat nach einem der Ansprüche 1, 3, 5, 7, 9 und 11, gekennzeichnet durch parenterale Verabreichung mit einer Dosis von 0,3 bis 2 mg/kg pro Tag, bezogen auf das Gewicht eines erwachsenen Patienten.

14. Verwendung von Caroverin und/oder pharmazeutisch verträglichen Caroverinsalzen, insbesondere Caroverinfumarat nach einem der Ansprüche 1, 3, 5, 7, 9 und 11, gekennzeichnet durch orale Verabreichung mit einer Dosis von 2 bis 10 mg/kg pro Tag, bezogen auf das Gewicht eines erwachsenen Patienten.

15. Verwendung von Caroverin und/oder pharmazeutisch verträglichen Caroverinsalzen, insbesondere Caroverinfumarat für die Behandlung von tachycarden Herzrhythmusstörungen.

16. Verwendung von Caroverin und/oder pharmazeutisch verträgichen Caroverinsalzen, insbesondere Caroverinfumarat zur Herstellung von pharmazeutischen Mitteln für die Behandlung von tachycarden Herzrhythmusstörungen.

17. Verwendung von Caroverin und/oder pharmazeutisch verträglichen Caroverinsalzen, insbesondere Caroverinfumarat für die Behandlung von hypertonen Zuständen.

18. Verwendung von Caroverin und/oder pharmazeutisch verträglichen Caroverinsalzen, insbesondere Caroverinfumarat zur Herstellung von pharmazeutischen Mitteln für die Behandlung von hypertonen Zuständen.

19. Verwendung von Caroverin und/oder pharmazeutisch verträglichen Caroverinsalzen, insbesondere Caroverinfumarat, nach Anspruch 15 oder 17, gekennzeichnet durch enterale oder parenterale Verabreichung in einer Dosis von 40 bis 400 mg, bezogen auf das Gewicht eines erwachsenen Patienten.

20. Verwendung von Caroverin und/oder einem pharmazeutisch verträglichen Caroverinsalz, insbesondere Caroverinfumarat, als Calcium-Blocker.

21. Verwendung von Caroverin und/oder einem pharmazeutisch verträglichen Caroverinsalz, insbesondere Caroverinfumarat, für die Herstellung von pharmazeutischen Mitteln als Calcium-Blocker.

22. Caroverinsalz mit einem sauren Xanthinderivat als Säurerest.

23. Caroverinsalz nach Anspruch 22, gekennzeichnet durch Theophyllin als Säurerest.

24. Caroverinsalz nach Anspruch 22, gekennzeichnet durch Coffein als Säurerest.

25. Caroverinsalz nach Anspruch 22, gekennzeichnet durch Theobromin als Säurerest.

26. Caroverinsalz, gekennzeichnet durch einen Nicotinsäurerest.

27. Verwendung von Caroverinsalzen nach einem oder mehreren der Ansprüche 22 bis 26 als pharmazeutischer Wirkstoff mit mindestens einer der folgenden Wirkungen bzw. Indikationen:

a) Thrombozyten-Aggregationshemmung

b) Erhöhung der Arteriendurchblutung

c) Erhöhung der Coronardurchblutung

d) Gefässerweiterung, Blutdrucksenkung, Bronchialdilatation

e) Herzfrequenzabsenkung

f) Spasmolyse

g) Diurese

h) Calcium-Antagonismus

i) Behandlung von ischämischen Herzkrankheiten

k) Behandlung von angina pectoris

m) Behandlung von Myocardinfarct

n) Behandlung von tachycarden Herzrhythmusstörungen

o) Behandlung von Hypertonie

p) Behandlung von Durchblutungsstörungen

q) Behandlung von cerebralen Durchblutungsstörungen

r) Behandlung, insbesondere prophylaktischer Behandlung, von Arteriosklerose

s) zentral wirkende Muskelrelaxation

28) Verwendung von Caroverinsalzen nach einem oder mehreren der Ansprüche 22 bis 26 zur Herstellung von pharmazeutischen Mitteln mit mindestens einer der folgenden Wirkungen bzw. Indikationen:

a) Thrombozyten-Aggregationshemmung

b) Erhöhung der Arteriendurchblutung

c) Erhöhung der Coronardurchblutung

d) Gefässerweiterung, Blutdrucksenkung, Bronchialdilatation

e) Herzfrequenzabsenkung

f) Spasmolyse

g) Diurese

h) Calcium-Antagonismus

i) Behandlung von ischämischen Herzkrankheiten

k) Behandlung von angina pectoris

m) Behandlung von Myocardinfarct

n) Behandlung von tachycarden Herzrhythmusstörungen

o) Behandlung von Hypertonie

p) Behandlung von Durchblutungsstörungen

q) Behandlung von cerebralen Durchblutungsstörungen

r) Behandlung, insbesondere prophylaktischer Behandlung, von Arteriosklerose

s) zentral wirkende Muskelrelaxation

29) Pharmazeutisches Mittel mit mindestens einer der folgenden Wirkungen bzw. Indikationen:

a) Thrombozyten-Aggregationshemmung

b) Erhöhung der Arteriendurchblutung

c) Erhöhung der Coronardurchblutung

d) Gefässerweiterung, Blutdrucksenkung, Bronchialdilatation

e) Herzfrequenzabsenkung

f) Spasmolyse

g) Diurese

h) Calcium-Antagonismus

i) Behandlung von ischämischen Herzkrankheiten

k) Behandlung von angina pectoris

m) Behandlung von Myocardinfarct

n) Behandlung von tachycarden Herzrhythmusstörungen

o) Behandlung von Hypertonie

p) Behandlung von Durchblutungsstörungen

q) Behandlung von cerebralen Durchblutungsstörungen

r) Behandlung, insbesondere prophylaktische Behandlung, von Arteriosklerose

s) zentral wirkende Muskelrelaxation

gekennzeichnet durch einen Gehalt an einem Caroverinsalz oder mehreren derselben mit folgendem Säuretest:

t) saures Xanthinderivat

u) Theophyllin

v) Coffein

w) Theombrin

x) Nikotinsäure

30) Pharmazeutisches Mittel mit blutdruckabsenkender und/oder diuretischer Wirkung, gekennzeichnet durch einen Gehalt an mindestens einem der folgenden Caroverinsalze:

a) Caroverin-Theophyllinat

b) Caroverin-Coffeinat

C) Caroverin-Theobrominat.

31) Pharmazeutisches Mittel mit thrombozyten-aggregationshemmender und/oder blutdruckabsenkender und/oder diuretischer Wirkung, gekennzeichnet durch einen Gehalt an Caroverin-Nicotinat.

32) Pharmazeutisches Mittel nach einem der Ansprüche 29 bis 31, gekennzeichnet durch eine Trägersubstanz, enthaltend wenigstens einen der folgenden Bestandteile:

a) Gelatine
b) Saccharide
c) Stärke
d) Fettsäure
e) Fettsäuresalze, insbesondere Calcium- und/oder Magnesiumsalze
f) Talcum
g) vegetabiles Oel
h) Alkohol, insbesondere Stearyl- und/oder Benzylalkohol
i) Polyalkylglykole
k) Gummi
l) Zellulosederivate
m) Polyvenylpyrolidon (PVP)
n) Casein-Formaldehyd
o) Metacrylsäurederivate.

33) Verwendung von Caroverin und/oder pharmazeutisch verträglichen Caroverinsalzen, insbesondere Caroverinfumarat, als zentral wirkendes Muskelrelaxans.

34) Verwendung von Caroverin und/oder pharmazeutisch verträglichen Caroverinsalzen, insbesondere Caroverinfumarat, für die Herstellung von pharmazeutischen Mitteln mit zentral muskelrelaxierender Wirkung.

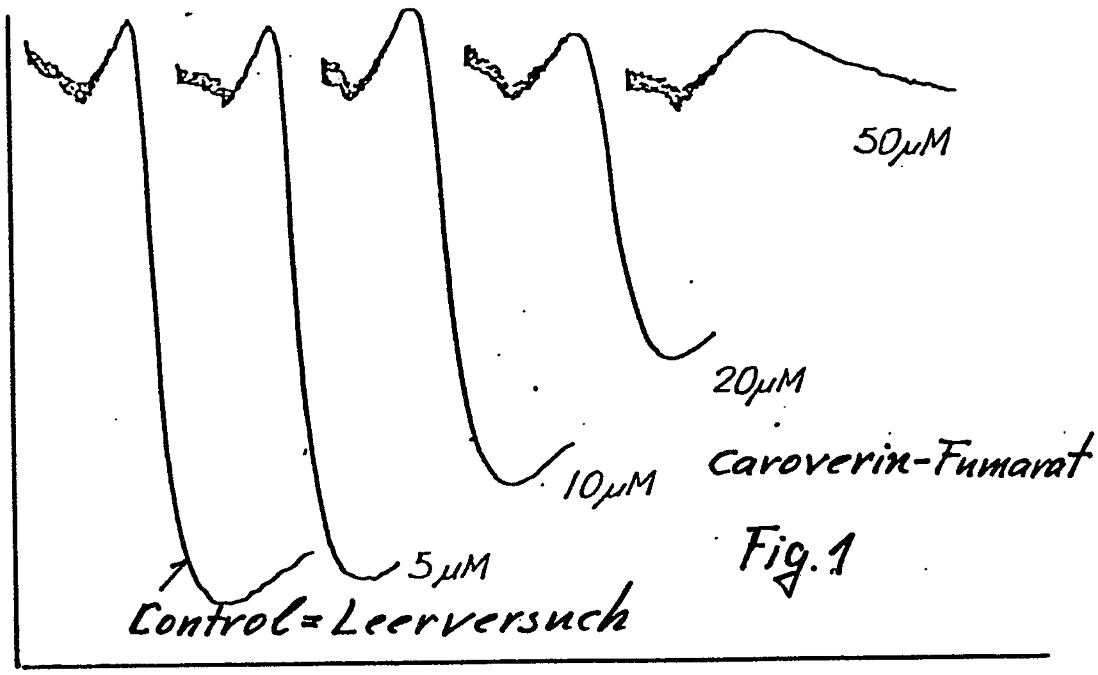


Fig. 1

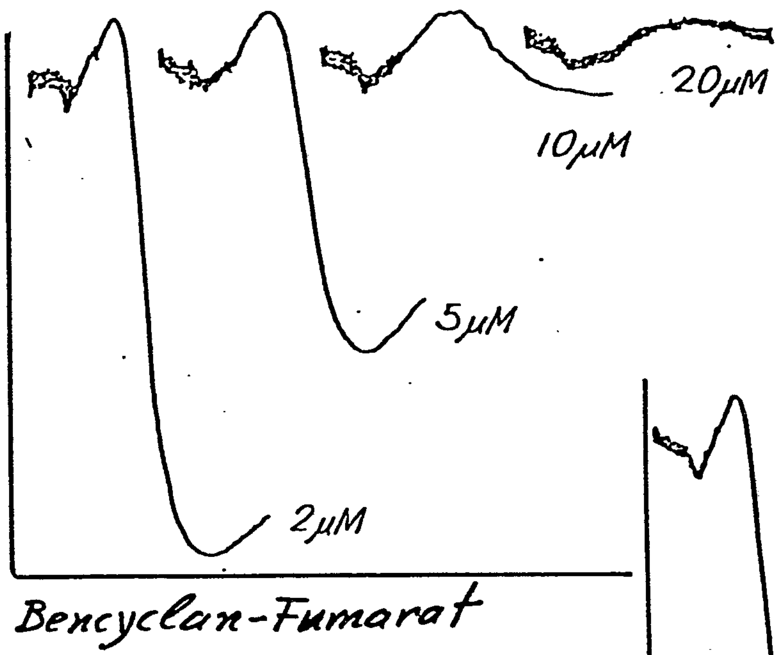


Bencyclan-Fumarat

Fig. 2

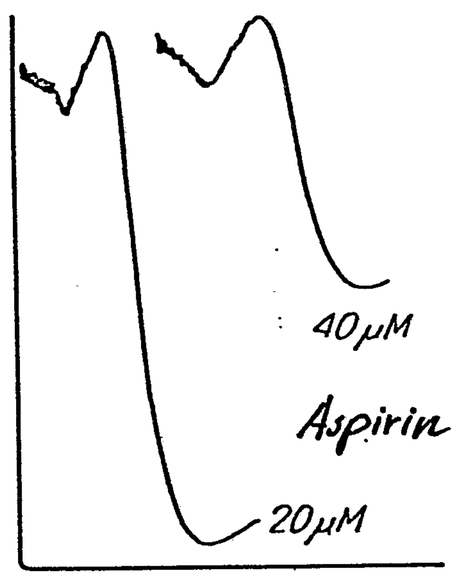


Fig. 3

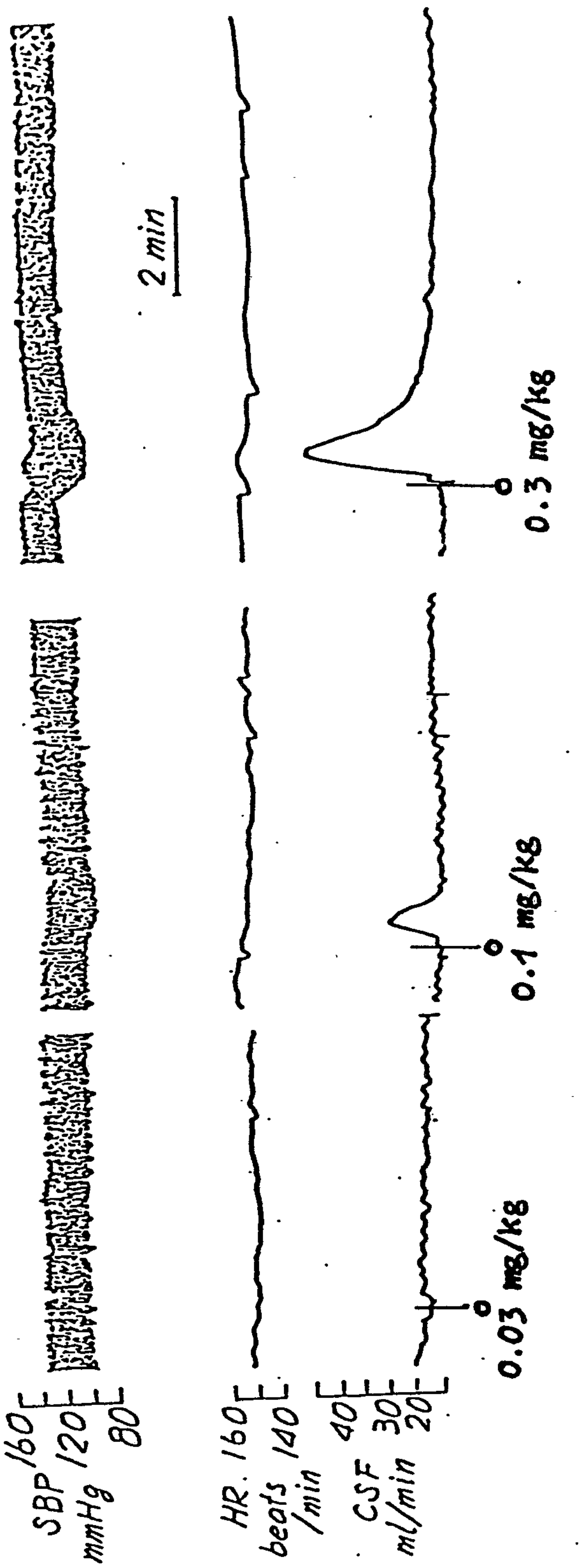
SBP mmHg
160
120
80
HR. beats /min
160
140
CSF ml/min
40
30
20
2 min
0.03 mg/kg
0.1 mg/kg
0.3 mg/kg

Fig. 4

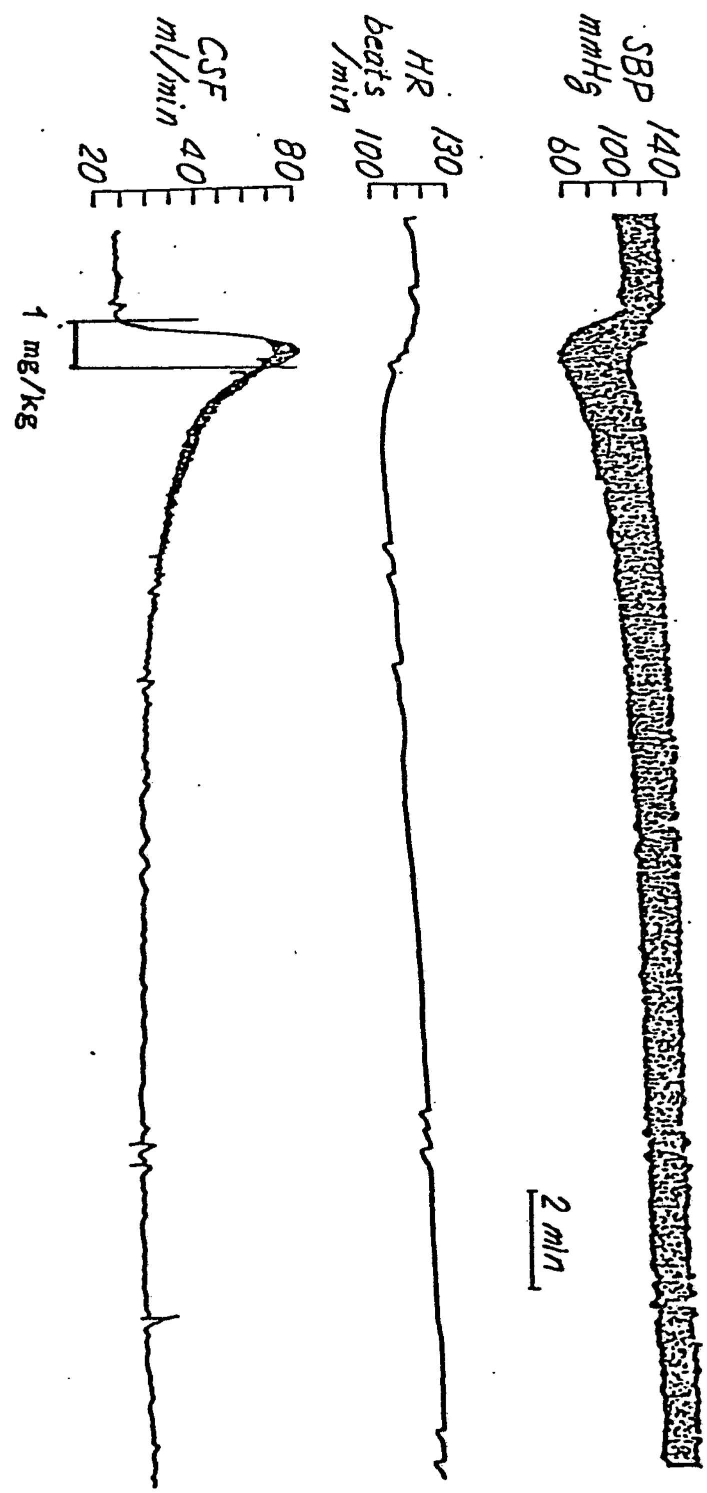

SBP mmHg
140
100
60
HR beats /min
130
100
CSF ml/min
80
40
20
1 mg/kg
2 min


Fig. 5

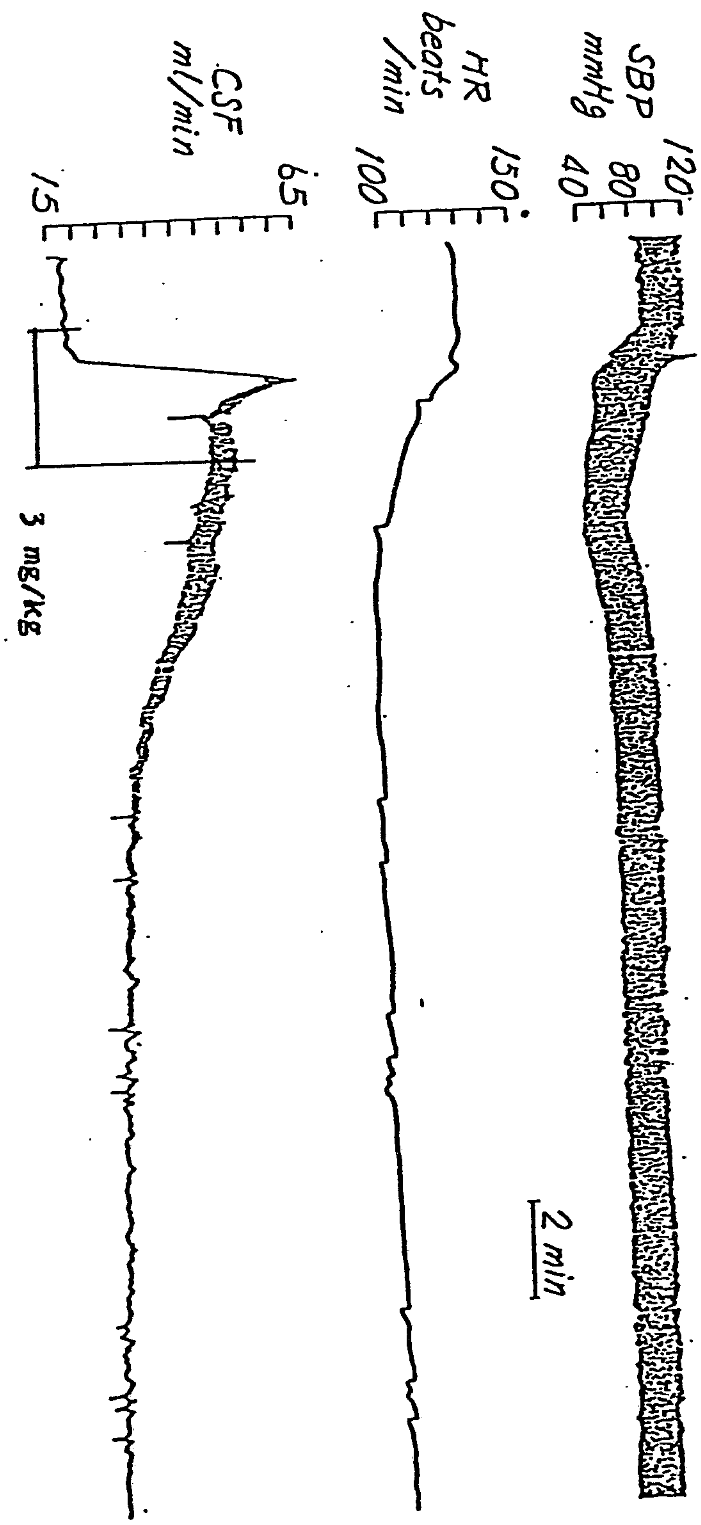
SBP
mmHg
120
80
40
HR
beats
/min
150
100
CSF
ml/min
65
15
3 mg/kg
2 min

Fig. 6

FIG. 7

Tabelle I

| Substanz | Coronarfluss (ml/min.) | | Kontraktionsamplitude (mm) | |
|---|---|---|---|---|
| | Ausgangshöhe | Zunahme | Ausgangshöhe | Zunahme |
| **CAROVERIN** µg: | | | | |
| 1 | 7.8±0.60 (5) | +3.6±0.61 | 14.0±1.64 (5) | -0.6±0.24 |
| 10 | 7.9±0.55 (5) | +4.7±0.42 | 13.6±1.83 (5) | -0.8±0.37 |
| 100 | 8.1±0.67 (5) | +7.0±1.04 | 11.2±1.77 (5) | -4.0±0.84 |
| 1000 | 7.5±0.54 (5) | +8.7±1.38 | 10.0±1.52 (5) | -10.0±1.52 |

Tabelle II

| Substanz | Coronarfluss (ml/min.) | | Kontraktionsamplitude (mm) | |
|---|---|---|---|---|
| | Ausgangshöhe | Zunahme | Ausgangshöhe | Zunahme |
| **THEOPHYLLIN** µg: | | | | |
| 10 | 7.9±0.61 (5) | +1.7±0.48 | 13.8±1.83 (5) | +0.4±0.58 |
| 100 | 8.1±0.47 (5) | +2.8±0.35 | 12.0±2.02 (5) | +0.8±0.58 |
| 1000 | 8.4±0.81 (5) | +5.5±0.86 | 10.0±1.67 (5) | -0.6±1.21 |

Patient: A

Fig. 8

Tabelle III

| Min. | Frequenz/min.* | Blutdruck mm Hg | Injektionsmenge (mg) |
|---|---|---|---|
| 0 | 150 | 175/80 | |
| 1 | 125 | 155/70 | 60 |
| 2 | 120 | 150/60 | 120 |
| 3 | 113 | 145/60 | 180 |
| 4 | 116 | 140/55 | 200 |
| 5 | 115 | 140/55 | |
| 6 | 111 | 145/60 | |
| 7 | 114 | 150/60 | |
| 8 | 113 | 160/60 | |
| 9 | 117 | 170/70 | |
| 10 | 120 | 175/70 | |

*mittlere Frequenz pro 10 QRS Aktionen.

Patient: B

Tabelle IV

Fig. 9

| Min. | Frequenz/min.* | Blutdruck mm Hg | Injektionsmenge (mg) |
|---|---|---|---|
| 0 | 71 | 160/80 | 0 |
| 1 | 59 | 120/60 | 80 |
| 2 | 64 | 110/50 | 160 |
| 3 | 65 | 100/50 | 200 |
| 4 | 61 | 105/60 | |
| 5 | 62 | 120/70 | |
| 6 | 61 | 130/60 | |
| 7 | 60 | 130/60 | |
| 8 | 48 | 135/70 | |
| 9 | 48 | 140/70 | |
| 10 | 51 | 130/60 | |
| 11 | 54 | 135/60 | |
| 12 | 50 | 135/60 | |
| 13 | 57 | 140/60 | |

*mittlere Frequenz pro 10 QRS Aktionen.

Patient: C

Tabelle V

Fig. 10

| Min. | Frequenz/min.* | Blutdruck mm Hg | Injektionsmenge (mg) |
|---|---|---|---|
| 0 | 90 | 130/70 | 0 |
| 1 | 87 | 120/70 | 80 |
| 2 | 82 | 90/60 | 140 |
| 3 | 88 | 90/60 | 200 |
| 4 | 78 | 90/70 | |
| 5 | 76 | 100/80 | |
| 6 | 69 | 110/80 | |
| 7 | 67 | 110/70 | |
| 8 | 77 | 120/90 | |
| 9 | 64 | 125/90 | |
| 10 | 65 | 130/80 | |
| 11 | 65 | 130/80 | |
| 12 | 68 | 140/90 | |

*mittlere Frequenz pro 10 QRS Aktionen.

Patient: D

Tabelle VI

Fig. 11

| Min. | Frequenz/min.* | Blutdruck mm Hg | Injektionsmenge (mg) |
|---|---|---|---|
| 0 | 77 | 190/90 | 0 |
| 1 | 63 | 130/70 | 80 |
| 2 | 59 | 130/70 | 140 |
| 3 | 60 | 120/70 | 200 |
| 4 | 64 | 130/70 | |
| 5 | 57 | 140/80 | |
| 6 | 54 | 140/80 | |
| 7 | 53 | 150/80 | |
| 8 | 51 | 150/80 | |
| 9 | 57 | 150/80 | |
| 10 | 50 | 150/80 | |

*mittlere Frequenz pro 10 QRS Aktionen.

FIG. 11 a Herzfrequenzen (Mittelwerte ± St.fehler) vor, während und nach Applikation von CAROVERIN bei Behandlungsgruppe A: 6 Normalpersonen

B: 6 Patienten mit koronarer Herzkrankheit

C: 6 Patienten mit krankem Reizleitungssystem

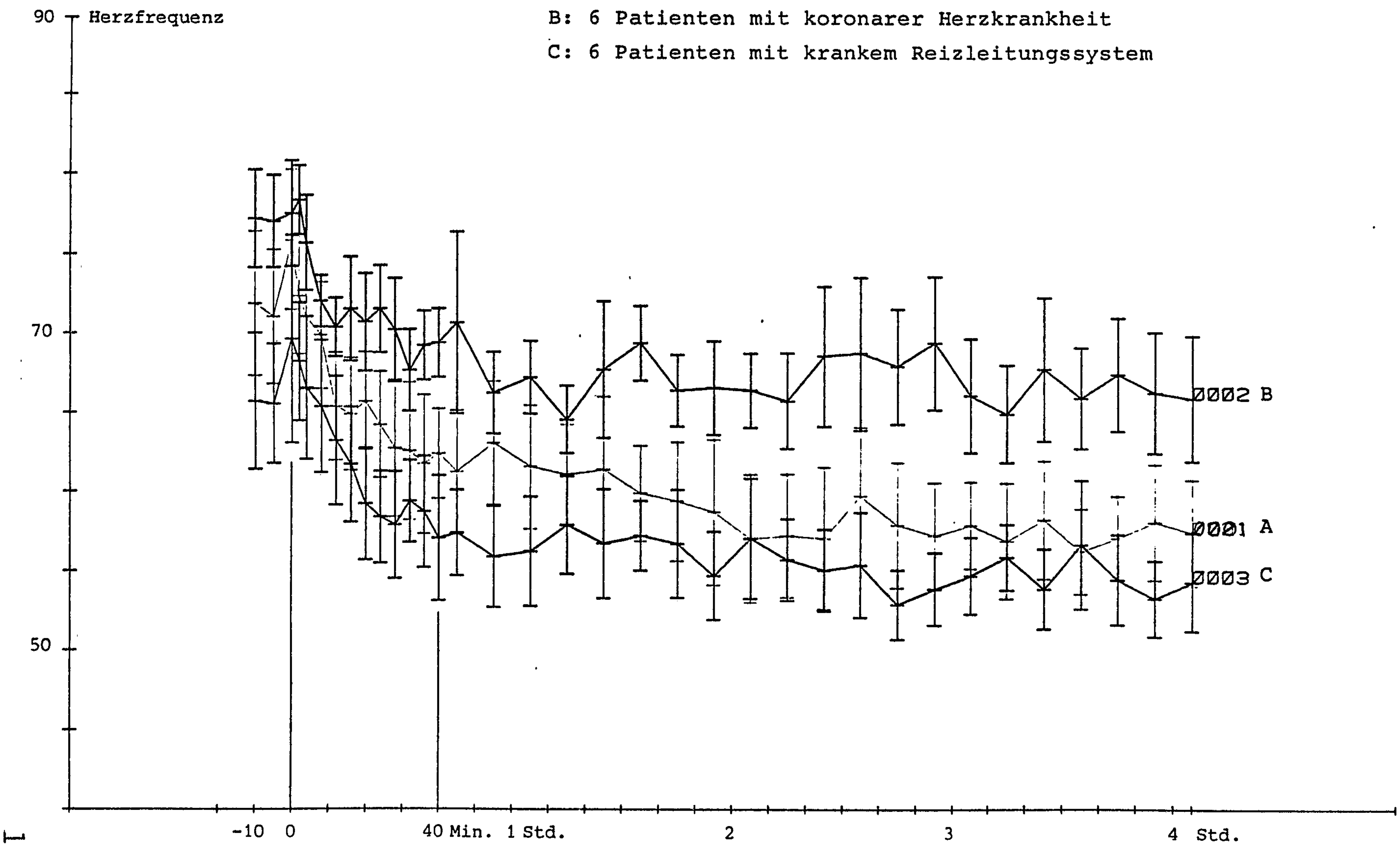

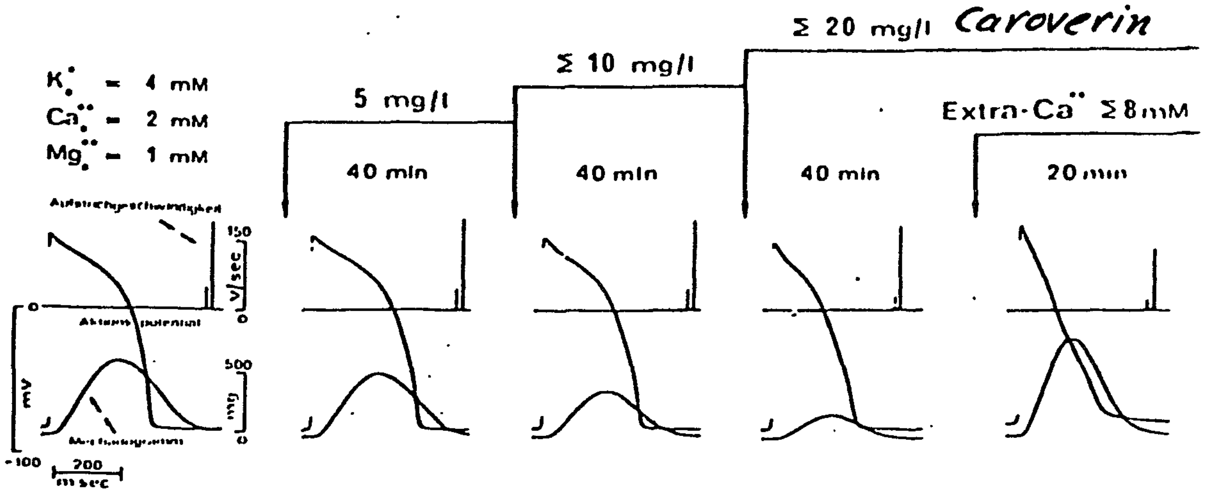


*Fig. 12*

Meerschweinchen - Papillarmuskel
Reizfrequenz : 30 min⁻¹
Temperatur : 30 °C

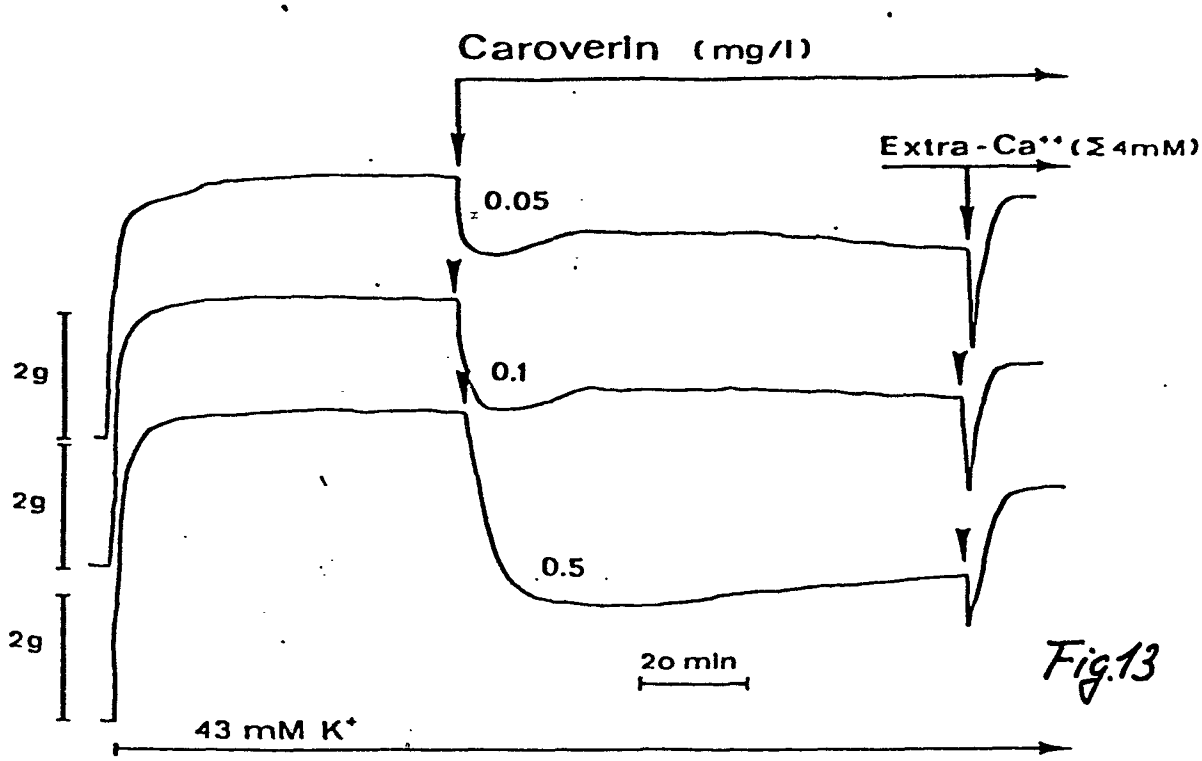


*Fig. 13*

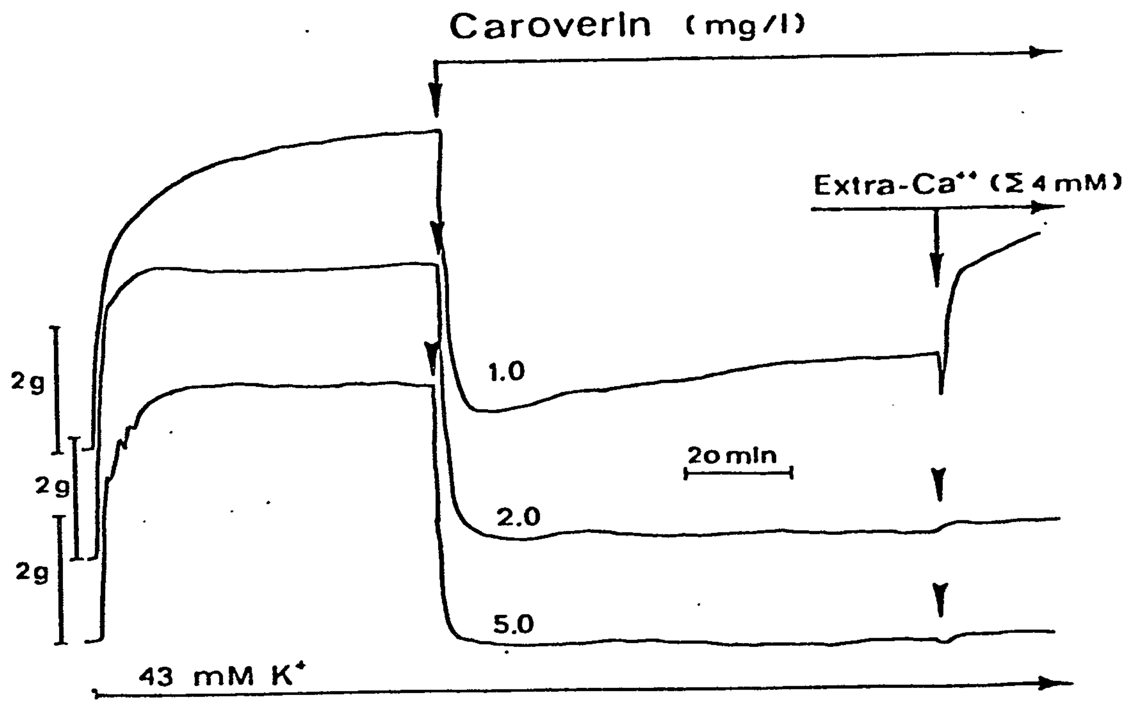
Caroverin (mg/l)
Extra-Ca⁺⁺ (Σ 4 mM)
2g
2g
2g
1.0
20 min
2.0
5.0
43 mM K⁺

Fig. 14

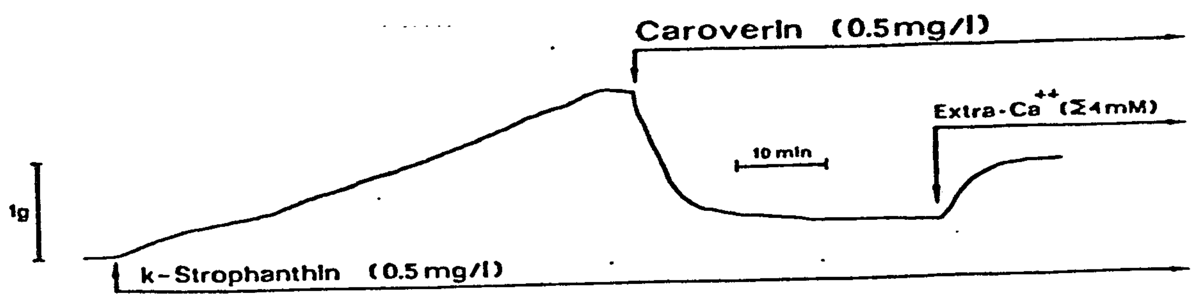
Caroverin (0.5 mg/l)
Extra-Ca⁺⁺ (Σ4 mM)
10 min
1g
k-Strophanthin (0.5 mg/l)

Fig. 15

Fig. 16

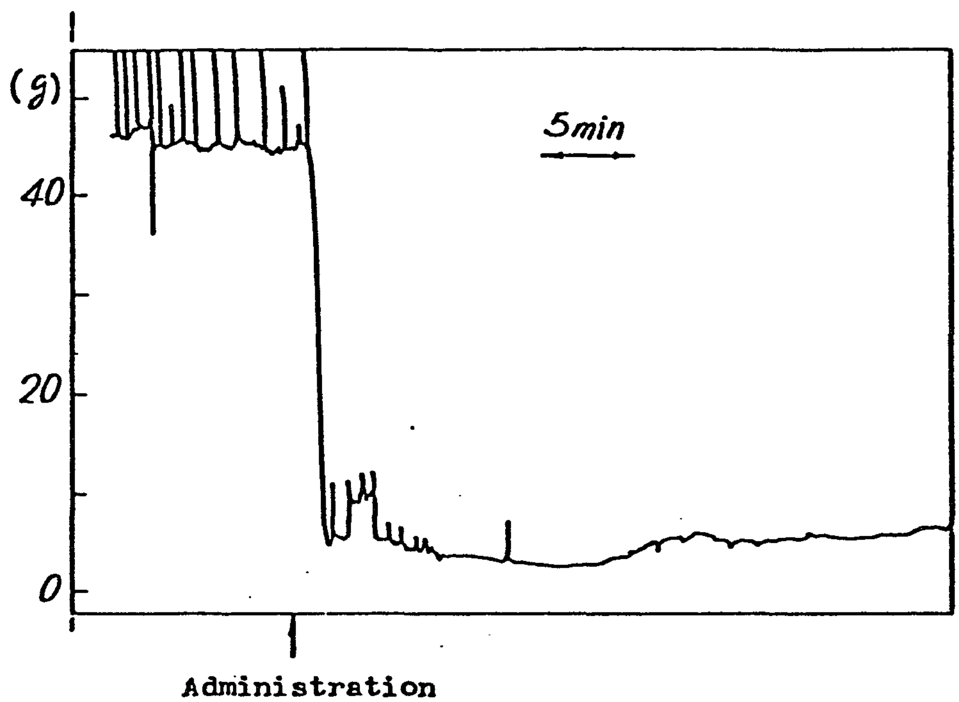

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung
EP 80 10 8008

## EINSCHLÄGIGE DOKUMENTE

| Kategorie | Kennzeichnung des Dokuments mit Angabe, soweit erforderlich, der maßgeblichen Teile | betrifft Anspruch |
| --- | --- | --- |
| X | DE - A - 2 521 905 (MEDICHEMIE)<br>* Insgesamt *<br>--- | 4-20, 33,34 |
| X | DE - A - 1 804 328 (DONAU)<br>* Beispiel 4; Seiten 1,2 *<br>--- | 4-20, 33,34 |
| X | FR - A - 1 393 957 (DONAU)<br>* Beispiel 2; Seite 1 *<br>& DE - B - 1 189 552<br>--- | 4-20, 33,34 |
| X | CHEMICAL ABSTRACTS, Band 76, Heft 19, 8. Mai 1972, Zusammenfassung 107959r, Seite 24, COLUMBUS, OHIO (US)<br>H. OZAWA et al.: "Pharmacological effects of 1-diethylamino-ethyl-3-(p-methoxybenzyl)-2-quinoxalone"<br>& Oyo Yakuri 1970, 4(2) 233-40<br>* Insgesamt *<br>--- | 4-20, 33,34 |

./..

KLASSIFIKATION DER ANMELDUNG (Int. Cl.³)

C 07 D 241/44
473/08
473/10
473/12
A 61 K 31/495
31/52

RECHERCHIERTE SACHGEBIETE (Int. Cl.³)

A 61 K 31/52
31/495
C 07 D 241/44
473/08
473/10
473/12

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 4-34
Unvollständig recherchierte Patentansprüche:
Nicht recherchierte Patentansprüche: 1-3
Grund für die Beschränkung der Recherche: Verfahren zur chirurgischen oder therapeutischen Behandlung des menschlichen oder tierischen Körpers. (Siehe Art. 52(4) des Europäischen Patentübereinkommens).

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
| --- | --- | --- |
| Den Haag | 26.03.1981 | NUYTS |

EPA Form 1505.1 06.78

Europäisches Patentamt

# EUROPÄISCHER TEILRECHERCHENBERICHT

Nummer der Anmeldung
EP 80 10 8008



| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| X | CHEMICAL ABSTRACTS, Band 83, Heft 9, 1. September 1975, Seiten 63-64, Zusammenfassung 71860e, COLUMBUS, OHIO (US)<br>S. HAYASHI: "Antispasmodic action of 1-diethylaminoethyl-3(p-methoxybenzyl)-2-quinoxalone (P 201-1) and its inhibitory effect on cyclic 3',5'-nucleotide phosphodiesterase activity"<br>& Chem. Pharm. Bull. 1975, 23(4) 810-16<br>* Insgesamt *<br>--------- | 4-20, 33,34 | RECHERCHIERTE SACHGEBIETE (Int. Cl.3) |